# EUROPEAN PATENT APPLICATION

(11) **EP 4 617 648 A1**
(43) Date of publication of application: **17.09.2025**
(21) Application number: 25155631.2
(22) Date of filing: 03.02.2025
(51) Int. Cl.: G01N 27/18, G01N 33/00

(54) **SENSOR**

(30) Priority: 12.03.2024 JP 2024038242
(71) Applicant: Kabushiki Kaisha Toshiba, Minato-ku Tokyo (JP)
(72) Inventor: YAMAZAKI, Yui, Tokyo (JP); HAMASAKI, Hiroshi, Tokyo (JP); AKIMOTO, Yosuke, Tokyo (JP)
(74) Representative: Marks & Clerk LLP

(57) **Abstract**

According to one embodiment, a sensor includes an element section, and a controller. The element section including a first element including a first detection section, a second element including a second detection section, and a third element including a third detection section. The first detection section includes a first resistance member and a first conductive member. The second detection section includes a second resistance member and a second conductive member. The third detection section includes a third resistance member. The controller is configured to perform a first operation, a second operation, and a third operation.

## Description

### FIELD

Embodiments described herein relate generally to a sensor.

### BACKGROUND

For example, there are sensors using MEMS (Micro Electro Mechanical Systems) elements. It is desired to improve the characteristics of sensors.

### BRIEF DESCRIPTION OF THE DRAWINGS

FIGS. 1A to 1C are schematic diagrams illustrating a sensor according to a first embodiment;
FIG. 2 is a schematic cross-sectional view illustrating the sensor according to the first embodiment;
FIGS. 3A and 3B are schematic plane views illustrating the sensor according to the first embodiment;
FIGS. 4A and 4B are schematic plane views illustrating the sensor according to the first embodiment; and
FIGS. 5A and 5B are schematic plane views illustrating the sensor according to the first embodiment.

### DETAILED DESCRIPTION

According to one embodiment, a sensor includes an element section, and a controller. The element section including a first element including a first detection section, a second element including a second detection section, and a third element including a third detection section. The first detection section includes a first resistance member and a first conductive member. The second detection section includes a second resistance member and a second conductive member. The third detection section includes a third resistance member. The controller is configured to perform a first operation, a second operation, and a third operation. In the first operation, the controller is configured to apply a first voltage to the first conductive member, not to apply the first voltage to the second conductive member, and to detect a first value corresponding to a first difference between a first electrical resistance of the first resistive member and a second electrical resistance of the second resistive member. In the second operation, the controller is configured to apply a second voltage to the first conductive member and to detect a second value corresponding to a second difference between the first electrical resistance and a third electrical resistance of the third resistive member. In the third operation, the controller is configured to apply a third voltage to the second conductive member and to detect a third value corresponding to a third difference between the second electrical resistance and the third electrical resistance.

Various embodiments are described below with reference to the accompanying drawings.

The drawings are schematic and conceptual; and the relationships between the thickness and width of portions, the proportions of sizes among portions, etc., are not necessarily the same as the actual values. The dimensions and proportions may be illustrated differently among drawings, even for identical portions.

In the specification and drawings, components similar to those described previously or illustrated in an antecedent drawing are marked with like reference numerals, and a detailed description is omitted as appropriate.

### (First Embodiment)

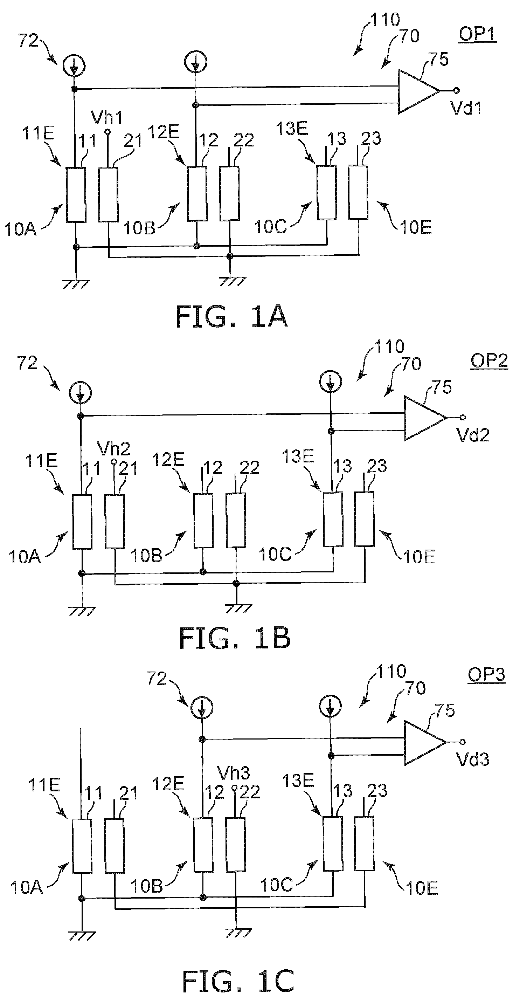

FIGS. 1A to 1C are schematic diagrams illustrating a sensor according to a first embodiment.

As shown in FIGS. 1A to 1C, a sensor 110 according to the embodiment includes an element section 10E and a controller 70. The element section 10E includes a first element 10A, a second element 10B, and a third element 10C.

The first element 10A includes a first detection section 11E. The second element 10B includes a second detection section 12E. The third element 10C includes a third detection section 13E.

The first detection section 11E includes a first resistance member 11 and a first conductive member 21. The second detection section 12E includes a second resistance member 12 and a second conductive member 22. The third detection section 13E includes a third resistance member 13. The third detection section 13E may include a third conductive member 23.

The controller 70 is configured to perform a first operation OP1, a second operation OP2, and a third operation OP3.

As shown in FIG.1A, in the first operation OP1, the controller 70 applies a first voltage Vh1 to the first conductive member 21 and does not apply the first voltage Vh1 to the second conductive member 22. In the first operation OP1, the controller 70 detects a first value Vd1 corresponding to a first difference Δ1 between a first electrical resistance R1 of the first resistance member 11 and a second electrical resistance R2 of the second resistance member 12. The controller 70 may output the first value Vd1.

As shown in FIG.1B, in the second operation OP2, the controller 70 applies a second voltage Vh2 to the first conductive member 21 and detects a second value Vd2 corresponding to a second difference Δ2 between the first electrical resistance R1 of the first resistance member 11 and a third electrical resistance R3 of the third resistance member 13. The controller 70 may output the second value Vd2. When the third detection section 13E includes the third conductive member 23, in the second operation OP2, the controller 70 does not apply the second voltage Vh2 to the third conductive member 23.

As shown in FIG.1C, in the third operation OP3, the controller 70 applies a third voltage Vh3 to the second conductive member 22 and detects a third value Vd3 corresponding to a third difference Δ3 between the second electrical resistance R2 of the second resistance member 12 and the third electrical resistance R3 of the third resistance member 13. The controller 70 may output the third value Vd3.

In the first operation OP1, by the first voltage Vh1 being supplied to the first conductive member 21, the temperature of the first conductive member 21 rises. As a result, the temperature of the first detection section 11E rises, and the temperature of the first resistance member 11 rises. The heat dissipation in the first detection section 11E changes according to the detection target (for example, the detection target gas) around the element section 10E. The temperature of the first detection section 11E and the temperature of the first resistance member 11 change according to the detection target around the element section 10E. By detecting the first electrical resistance R1 of the first resistance member 11, information on the detection target is obtained.

For example, in the first operation OP1, the second detection section 12E functions as a reference element. In the first operation OP1, by detecting the first difference Δ1 between the first electrical resistance R1 of the first resistance member 11 and the second electrical resistance R2 of the second resistance member 12, for example, the influence of a change in the ambient temperature or the like can be suppressed. Appropriate corrections are made.

In the second operation OP2, by the second voltage Vh2 being supplied to the first conductive member 21, the temperature of the first conductive member 21 rises. As a result, the temperature of the first detection section 11E rises, and the temperature of the first resistance member 11 rises. In the second operation OP2, information on the detection target is obtained by detecting the first electrical resistance R1 of the first resistance member 11. For example, in the second operation OP2, the third detection section 13E functions as another reference element. In the second operation OP2, by detecting the second difference Δ2 between the first electrical resistance R1 of the first resistance member 11 and the third electrical resistance R3 of the third resistance member 13, for example, the influence of a change in the ambient temperature or the like can be suppressed. Appropriate corrections are made.

In the third operation OP3, by the third voltage Vh3 being applied to the second conductive member 22, the temperature of the second conductive member 22 rises. As a result, the temperature of the second detection section 12E rises, and the temperature of the second resistance member 12 rises. In the third operation OP3, information on the detection target is obtained by detecting the second electrical resistance R2 of the second resistance member 12. In the third operation OP3, the third detection section 13E functions as a reference element. In the third operation OP3, by detecting the third difference Δ3 between the second electrical resistance R2 of the second resistance member 12 and the third electrical resistance R3 of the third resistance member 13, for example, the influence of a change in the ambient temperature or the like can be suppressed. Appropriate corrections are made.

As described above, in the first operation OP1, the first detection section 11E functions as a detection element, and the second detection section 12E functions as a reference element. In the second operation OP2, the first detection section 11E functions as a detection element, and the third detection section 13E functions as a reference element. In the third operation OP3, the second detection section 12E functions as a detection element, and the third detection section 13E functions as a reference element.

In a reference example, first to fourth elements are provided. The first element functions as a detection element and the second element functions as a reference element. The third element functions as a detection element, and the fourth element functions as a reference element. The four elements make it difficult to miniaturize the sensor.

In the embodiment, the second detection section 12E, which functions as a reference element in the first operation OP1, functions as a detection element in the third operation OP3. Highly accurate correction can be performed with fewer elements. Highly accurate detection becomes possible even with a small sensor. In embodiments, a sensor with improved characteristics can be provided.

In the embodiment, in the first operation OP1, the first electrical resistance R1 corresponds to the detection target around the element section 10E. The change rate of the first electrical resistance R1 with respect to the detection target in the first operation OP1 is higher than the change rate of the second electrical resistance R2 with respect to the detection target in the first operation OP1.

In the second operation OP2, the first electrical resistance R1 responds to the detection target around the element section 10E. The change rate of the first electrical resistance R1 with respect to the detection target in the second operation OP2 is higher than the change rate of the third electrical resistance R3 with respect to the detection target in the second operation OP2.

In the third operation OP3, the second electrical resistance R2 responds to the detection target around the element section 10E. The change rate of the second electrical resistance R2 with respect to the detection target in the third operation OP3 is higher than the change rate of the third electrical resistance R3 with respect to the detection target in the third operation OP3.

In the embodiment, the controller 70 may be configured to output a fourth value obtained by calculation using the first value Vd1, the second value Vd2, and the third value Vd3. The fourth value may include multiple values. The fourth value may include a value corresponding to each concentration of a plurality of types of detection substances included in the detection target.

For example, the plurality of detection substances may include at least two selected from the group consisting of carbon dioxide, hydrogen, nitrogen, and carbon monoxide. For example, a relational expression representing the relationship between the concentration of each of the plurality of types of detection substances and the heat dissipation property (detected temperature, that is, detected electrical resistance) can be derived. The controller 70 can derive conditions that satisfy the plurality of relational expressions by calculation using the first value Vd1, the second value Vd2, and the third value Vd3. The conditions that satisfy the plurality of relational expressions correspond to the respective concentrations of the plurality of types of detection substances included in the detection target.

The first difference Δ1, the second difference Δ2, and the third difference Δ3 may be output from a differential circuit 75, for example. The differential circuit 75 may be included in the controller 70.

As shown in FIG. 1A, in the first operation OP1, a detection current is supplied from a current source 72 to the first resistance member 11 and the second resistance member 12. As shown in FIG. 1B, in the second operation OP2, the detection current is supplied from the current source 72 to the first resistance member 11 and the third resistance member 13. As shown in FIG. 1C, in the third operation OP3, the detection current is supplied from the current source 72 to the second resistance member 12 and the third resistance member 13. The current source 72 may be included in controller 70.

In the embodiment, the first voltage Vh1 may be different from the second voltage Vh2. By applying different voltages, the first detection section 11E rises to different temperatures. The reference voltage in the differential detection of the first operation OP1 is different from the reference voltage in the differential detection of the second operation OP2. The detection targets can be detected with higher accuracy. For example, multiple types of detection targets can be detected.

For example, a first power consumption is calculated from the first voltage Vh1 and the first conductive member resistance value of the first conductive member 21. A second power consumption is calculated from the second voltage Vh2 and the first conductive member resistance value of the first conductive member 21. For example, the first power consumption is smaller than the second power consumption. The difference between the first power consumption and the second power consumption is defined as a first power consumption difference. The ratio of the first absolute value of the first power consumption difference to the first power consumption is preferably 0.4 or more. Thereby, it becomes easier to detect multiple types of detection targets with higher accuracy. The ratio may be, for example, 1.5 or less.

For example, the first power consumption may be between the third power consumption and the second power consumption. As already explained, the first power consumption is calculated from the first voltage Vh1 and the first conductive member resistance value of the first conductive member 21. The second power consumption is calculated from the second voltage Vh2 and the second conductive member resistance value of the first conductive member 21. The third power consumption is calculated from the third voltage Vh3 and the second conductive member resistance value of the second conductive member 22. For example, the third power consumption may be smaller than the second power consumption. The first power consumption may be between the third power consumption and the second power consumption.

For example, a third electrical resistance value of the third resistance member 13 at the first temperature T1 may be different from a first electrical resistance value of the first resistance member 11 at the first temperature T1. The third electrical resistance value may be different from a second electrical resistance value of the second resistance member 12 at the first temperature T1.

As described above, in the first operation OP1, the first voltage Vh1 is supplied to the first conductive member 21. The temperature of the first detection section 11E when the first voltage Vh1 is supplied to the first conductive member 21 is defined as a first increased temperature. In the second operation OP2, the second voltage Vh2 is applied to the first conductive member 21. The temperature of the first detection section 11E when the second voltage Vh2 is applied to the first conductive member 21 is defined as a second increased temperature. In the third operation OP3, the third voltage Vh3 is applied to the second conductive member 22. The temperature of the second detection section 12E when the third voltage Vh3 is applied to the second conductive member 22 is referred to as a third increased temperature. The first increased temperature may correspond to the temperature of the first resistance member 11 in the first operation OP1. The second increased temperature may correspond to the temperature of the first resistance member 11 in the second operation OP2. The third increased temperature may correspond to the temperature of the second resistance member 12 in the third operation OP3.

The first temperature T1 described above is lower than the temperature (first increased temperature) of the first detection section 11E in the first operation OP1. The first temperature T1 is lower than the temperature (second increased temperature) of the first detection section 11E in the second operation OP2. The first temperature T1 is lower than the temperature (third increased temperature) of the second detection section 12E in the third operation OP3. The first temperature T1 may be, for example, room temperature. The first temperature T1 may be, for example, not less than -10°C and not more than +80°C.

An absolute value of a difference between the first increased temperature and the second increased temperature is, for example, 30°C or more. An absolute value of a difference between the first increased temperature and the third increased temperature is, for example, 30°C or more. An absolute value of a difference between the second increased temperature and the third increased temperature is, for example, 30°C or more. By such a temperature difference, for example, values corresponding to the concentrations of a plurality of types of detection substances contained in the detection target are easily derived with high accuracy.

In one example, the third increased temperature is lower than the second increased temperature. For example, the first increased temperature may be between the third increased temperature and the second increased temperature.

For example, an absolute value of a difference between the third electrical resistance value at the first temperature T1 and the first electrical resistance value at the first temperature T1 may be larger than an absolute value of a difference between the first electrical resistance value at the first temperature T1 and the second electrical resistance value at the first temperature T1.

In the embodiment, the third detection section 13E may further include a third conductive member 23 (see FIGS. 1A to 1C). In the second operation OP2, the controller 70 does not need to apply the second voltage Vh2 to the third conductive member 23. In the third operation OP3, the controller 70 does not need to apply the third voltage Vh3 to the third conductive member 23.

Hereinafter, examples of the first element 10A, the second element 10B, and the third element 10C will be described.

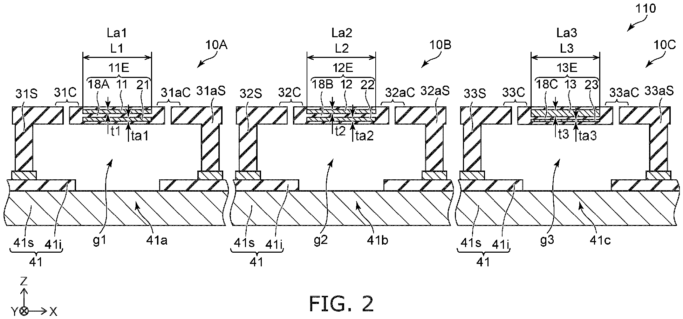

FIG. 2 is a schematic cross-sectional view illustrating the sensor according to the first embodiment.

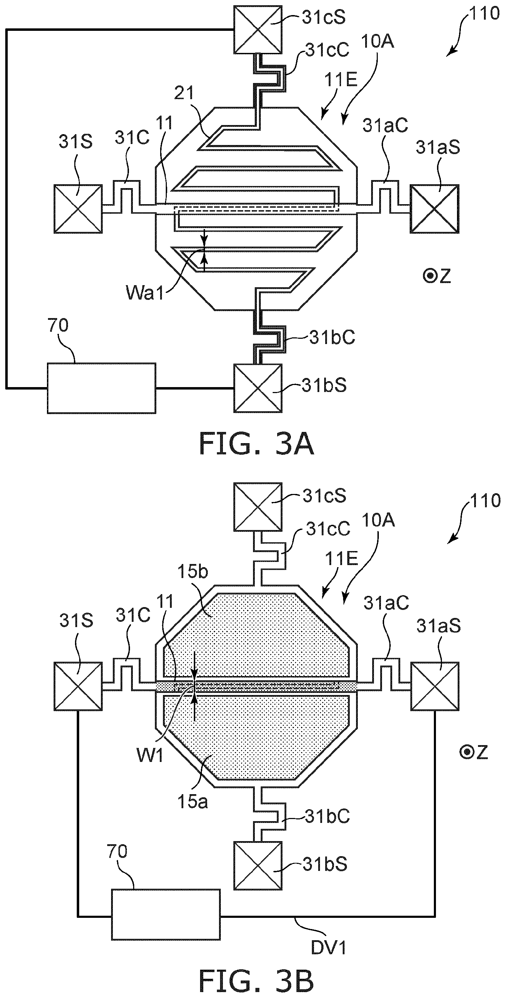

FIGS. 3A, 3B, 4A, 4B, 5A, and 5B are schematic plane views illustrating the sensor according to the first embodiment.

As shown in FIG. 2, the first element 10A may include a first base region 41a, a first fixed portion 31S, and a first support portion 31C. The first fixed portion 31S is fixed to a part of the first base region 41a. The first support portion 31C is supported by the first fixed portion 31S. The first support portion 31C supports the first detection section 11E. A first gap g1 is provided between another part of the first base region 41a and the first detection section 11E.

In this example, the first element 10A further includes a first opposing fixed portion 31aS and a first opposing support portion 31aC. The first opposing fixed portion 31aS is fixed to another part of the first base region 41a. The first opposing support portion 31aC is supported by the first opposing fixed portion 31aS. The first opposing support portion 31aC supports the first detection section 11E. For example, the first detection section 11E is provided between the first support portion 31C and the first opposing support portion 31aC.

As shown in FIGS. 3A and 3B, the first element 10A may include a first cross fixed portion 31bS, a first cross support portion 31bC, a first cross opposing fixed portion 31cS, and a first cross opposing support portion 31cC. The first cross fixed portion 31bS is fixed to another part of the first base region 41a. The first cross support portion 31bC is supported by the first cross fixed portion 31bS. The first cross support portion 31bC supports the first detection section 11E.

The first cross opposing fixed portion 31cS is fixed to another part of the first base region 41a. The first cross opposing support portion 31cC is supported by the first cross opposing fixed portion 31cS. The first cross opposing support portion 31cC supports the first detection section 11E.

A direction from a part of the first base region 41a to the first fixed portion 31S is defined as a Z-axis direction (first direction). A direction from the first support portion 31C to the first opposing support portion 31aC crosses the Z-axis direction. A direction from the first cross support portion 31bC to the first cross opposing support portion 31cC crosses the Z-axis direction. A direction from the first cross support portion 31bC to the first cross opposing support portion 31cC crosses the direction from the first support portion 31C to the first opposing support portion 31aC.

At least one of the first support portion 31C, the first opposing support portion 31aC, the first cross support portion 31bC, and the first cross opposing support portion 31cC may have a meandering structure.

As illustrated in FIG.2, the second element 10B may include a second base region 41b, a second fixed portion 32S, and a second support portion 32C. The second fixed portion 32S is fixed to a part of the second base region 41b. The second support portion 32C is supported by the second fixed portion 32S. The second support portion 32C supports the second detection section 12E. A second gap g2 is provided between another part of the second base region 41b and the second detection section 12E.

In the example, the second element 10B further includes a second opposing fixed portion 32aS and a second opposing support portion 32aC. The second opposing fixed portion 32aS is fixed to another part of the second base region 41b. The second opposing support portion 32aC is supported by the second opposing fixed portion 32aS. The second opposing support portion 32aC supports the second detection section 12E. For example, the second detection section 12E is provided between the second support portion 32C and the second opposing support portion 32aC.

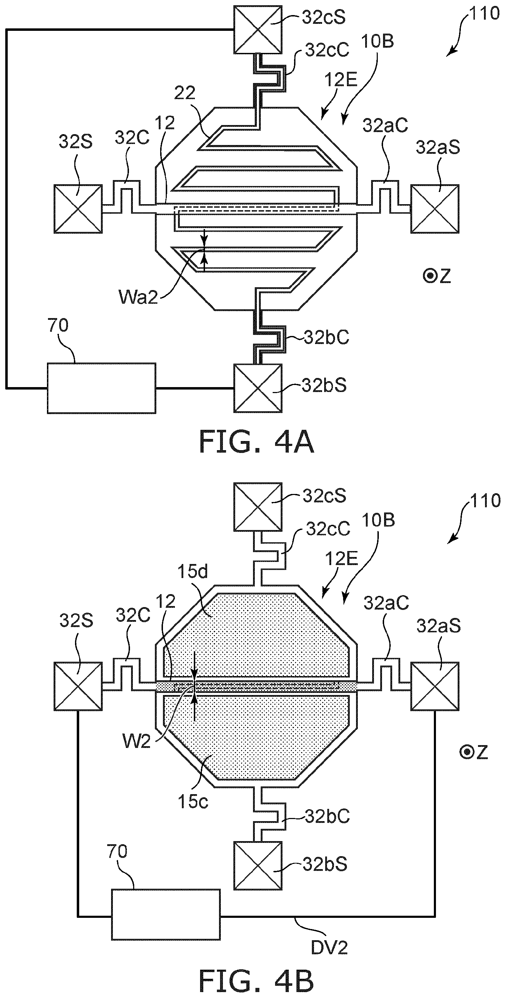

As shown in FIGS. 4A and 4B, the second element 10B may include a second cross fixed portion 32bS, a second cross support portion 32bC, a second cross opposing fixed portion 32cS, and a second cross opposing support portion 32cC. The second cross fixed portion 32bS is fixed to another part of the second base region 41b. The second cross support portion 32bC is supported by the second cross fixed portion 32bS. The second cross support portion 32bC supports the second detection section 12E.

The second cross opposing fixed portion 32cS is fixed to another part of the second base region 41b. The second cross opposing support portion 32cC is supported by the second cross opposing fixed portion 32cS. The second cross opposing support portion 32cC supports the second detection section 12E.

A direction from a part of the second base region 41b to the second fixed portion 32S may be along the Z-axis direction. A direction from the second support portion 32C to the second opposing support portion 32aC crosses the direction from the part of the second base region 41b to the second fixed portion 32S. A direction from the second cross support portion 32bC to the second cross opposing support portion 32cC crosses the direction from the part of the second base region 41b to the second fixed portion 32S. The direction from the second cross support portion 32bC to the second cross opposing support portion 32cC crosses the direction from the second support portion 32C to the second opposing support portion 32aC.

At least one of the second support portion 32C, the second opposing support portion 32aC, the second cross support portion 32bC, and the second cross opposing support portion 32cC may have a meander structure.

As illustrated in FIG.2, the third element 10C may include a third base region 41c, a third fixed portion 33S, and a third support portion 33C. The third fixed portion 33S is fixed to a part of the third base region 41c. The third support portion 33C is supported by the third fixed portion 33S. The third support portion 33C supports the third detection section 13E. A third gap g3 is provided between another part of the third base region 41c and the third detection section 13E.

In the example, the third element 10C further includes a third opposing fixed portion 33aS and a third opposing support portion 33aC. The third opposing fixed portion 33aS is fixed to another part of the third base region 41c. The third opposing support portion 33aC is supported by the third opposing fixed portion 33aS. The third opposing support portion 33aC supports the third detection section 13E. For example, the third detection section 13E is provided between the third support portion 33C and the third opposing support portion 33aC.

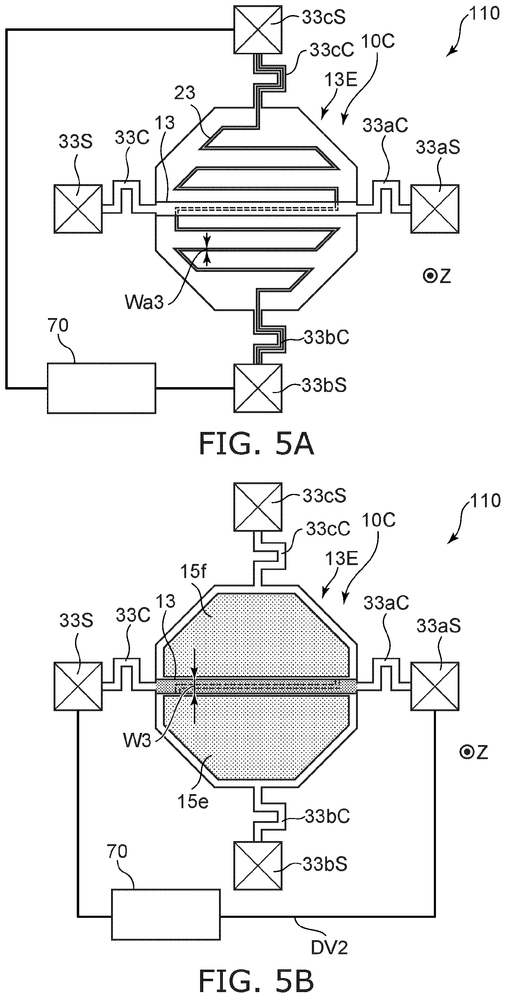

As shown in FIGS. 5A and 5B, the third element 10C may include a third cross fixed portion 33bS, a third cross support portion 33bC, a third cross opposing fixed portion 33cS, and a third cross opposing support portion 33cC. The third cross fixed portion 33bS is fixed to another part of the third base region 41c. The third cross support portion 33bC is supported by the third cross fixed portion 33bS. The third cross support portion 33bC supports the third detection section 13E.

The third cross opposing fixed portion 33cS is fixed to another part of the third base region 41c. The third cross opposing support portion 33cC is supported by the third cross opposing fixed portion 33cS. The third cross opposing support portion 33cC supports the third detection section 13E.

A direction from a part of the third base region 41c to the third fixed portion 33S may be along the Z-axis direction. A direction from the third support portion 33C to the third opposing support portion 33aC crosses the direction from the part of the third base region 41c to the third fixed portion 33S. A direction from the third cross support portion 33bC to the third cross opposing support portion 33cC crosses the direction from the part of the third base region 41c to the third fixed portion 33S. The direction from the third cross support portion 33bC to the third cross opposing support portion 33cC crosses the direction from the third support portion 33C to the third opposing support portion 33aC.

At least one of the third support portion 33C, the third opposing support portion 33aC, the third cross support portion 33bC, and the third cross opposing support portion 33cC may have a meandering structure.

As shown in FIG. 2, the first base region 41a is a part of the base 41. The second base region 41b may be another part of the base 41. The third base region 41c may be another part of the base 41. The first base region 41a may be one base 41, and the second base region 41b may be another base. The third base region 41c may be another base.

In this example, the base 41 may include a substrate 41s and an insulating layer 41i. The insulating layer 41i is provided on the substrate 41s. The substrate 41s includes a semiconductor substrate or the like. The substrate 41s may include a part of the circuit included in the controller 70.

As shown in FIG.2, the first detection section 11E may include a first insulating member 18A. The first insulating member 18A is provided around the first resistance member 11 and the first conductive member 21. The second detection section 12E may include a second insulating member 18B. The second insulating member 18B may be provided around the second resistance member 12 and the second conductive member 22. The third detection section 13E may include a third insulating member 18C. The third insulating member 18C may be provided around the third resistance member 13. The third insulating member 18C may be provided around the third conductive member 23.

For example, the first resistance member 11 and the second resistance member 12 may satisfy at least one of a first condition, a second condition, or a third condition. In the first condition, a first length L1 (see FIG.2) of the first resistance member 11 is different from a second length L2 (see FIG.2) of the second resistance member 12. In the second condition, a first width W1 (see FIG.3B) of the first resistance member 11 is different from a second width W2 (see FIG.4B) of the second resistance member 12. In the third condition, a first thickness t1 (see FIG.2) of the first resistance member 11 is different from a second thickness t2 of the second resistance member 12.

For example, the first resistance member 11 and the third resistance member 13 may satisfy at least one of a fourth condition, a fifth condition, or a sixth condition. In the fourth condition, the first length L1 of the first resistance member 11 is different from a third length L3 (see FIG.2) of the third resistance member 13. In the fifth condition, the first width W1 of the first resistance member 11 is different from a third width W3 of the third resistance member 13 (see FIG.5B). In the sixth condition, the first thickness t1 of the first resistance member 11 is different from a third thickness t3 (see FIG.2) of the third resistance member 13.

For example, the second resistance member 12 and the third resistance member 13 may satisfy at least one of a seventh condition, an eighth condition, or a ninth condition. In the seventh condition, the second length L2 of the second resistance member 12 is different from the third length L3 of the third resistance member 13. In the eighth condition, the second width W2 of the second resistance member 12 is different from the third width W3 of the third resistance member 13. In the ninth condition, the second thickness t2 of the second resistance member 12 is different from the third thickness t3 of the third resistance member 13.

The electrical resistance of the second conductive member 22 may be different from the electrical resistance of the first conductive member 21. The electrical resistance of the third conductive member 23 may be different from the electrical resistance of the first conductive member 21. The electrical resistance of the third conductive member 23 may be different from the electrical resistance of the second conductive member 22.

In the embodiment, for example, a second temperature T2 of the first resistance member 11 when the first electric power is supplied to the first conductive member 21 is higher than a first temperature T1. An absolute value of a difference between a first change rate of the first electrical resistance with respect to temperature at the first temperature T1 and a second change rate of the second electrical resistance with respect to temperature at the first temperature T1 is defined as a first change rate difference. An absolute value of a difference between a second change rate and a second temperature state change rate with respect to temperature of the first electrical resistance at the second temperature T2 is defined as a second change rate difference. In the embodiment, the second change rate difference may be smaller than the first change rate difference. Thereby, the detection result obtained from the first element 10A can be corrected with higher accuracy by the second element 10B. Detection with higher accuracy is possible. The second temperature T2 may be, for example, substantially the same as the first increased temperature.

In the embodiment, the third temperature T3 of the first resistance member 11 when the second electric power is supplied to the first conductive member 21 is higher than the first temperature T1. An absolute value of a difference between the first change rate and the third change rate of the third electrical resistance with respect to temperature at the first temperature T1 is defined as a third change rate difference. An absolute value of a difference between the third change rate and a third temperature state change rate of the first electrical resistance with respect to temperature at the third temperature T3 is defined as a fourth change rate difference. In the embodiment, the fourth change rate difference is smaller than the third change rate difference. Thereby, the detection result obtained from the first element 10A can be corrected with higher accuracy by the third element 10C. Detection with higher accuracy is possible. The third temperature T3 may be, for example, substantially the same as the second increased temperature.

For example, the first electrical resistance value of the first resistance member 11 at the first temperature T1 is different from the second electrical resistance value of the second resistance member 12 at the first temperature T1. For example, the first electrical resistance value may be different from the third electrical resistance value of the third resistance member 13 at the first temperature T1. For example, the second electrical resistance value may be different from the third electrical resistance value.

For example, the fourth temperature T4 of the second resistance member 12 when the third electric power is supplied to the second conductive member 22 is higher than the first temperature T1. An absolute value of a difference between the second change rate of the second electrical resistance with respect to temperature at the first temperature T1 and the third change rate of the third electrical resistance with respect to the temperature at the first temperature T1 is defined as a fifth change rate difference. An absolute value of a difference between a fourth temperature state change rate with respect to temperature of the second electrical resistance at the fourth temperature T4 and the third change rate is defined as a sixth change rate difference. In the embodiment, the sixth change rate difference may be smaller than the fifth change rate difference. Thereby, the detection result obtained from the second element 10B can be corrected with higher accuracy by the third element 10C. Detection with higher accuracy is possible. The fourth temperature T4 may be, for example, substantially the same as the third increased temperature.

The first conductive member 21 and the second conductive member 22 may satisfy at least one of a first conductive member condition, a second conductive member condition, or a third conductive member condition. In the first conductive member condition, a first conductive member length La1 (see FIG.2) of the first conductive member 21 is different from a second conductive member length La2 (see FIG.2) of the second conductive member 22. In the second conductive member condition, a first conductive member width Wa1 (see FIG.3A) of the first conductive member 21 is different from a second conductive member width Wa2 (see FIG.4A) of the second conductive member 22. In the third conductive member condition, a first conductive member thickness ta1 (see FIG.2) of the first conductive member 21 is different from a second conductive member thickness ta2 (see FIG.2) of the second conductive member 22.

For example, the first conductive member 21 and the third conductive member 23 may satisfy at least one of a fourth conductive member condition, a fifth conductive member condition, or a sixth conductive member condition. In the fourth conductive member condition, the first conductive member length La1 of the first conductive member 21 is different from a third conductive member length La3 (see FIG.2) of the third conductive member 23. In the fifth conductive member condition, the first conductive member width Wa1 of the first conductive member 21 is different from a third conductive member width Wa3 (see FIG.5A) of the third conductive member 23. In the sixth conductive member condition, the first conductive member thickness ta1 of the first conductive member 21 is different from a third conductive member thickness ta3 (see FIG.2) of the third conductive member 23.

For example, the second conductive member 22 and the third conductive member 23 may satisfy at least one of a seventh conductive member condition, an eighth conductive member condition, or a ninth conductive member condition. In the seventh conductive member condition, the second conductive member length La2 of the second conductive member 22 is different from the third conductive member length La3 of the third conductive member 23. In the eighth conductive member condition, the second conductive member width Wa2 of the second conductive member 22 is different from the third conductive member width Wa3 of the third conductive member 23. In the ninth conductive member condition, the second conductive member thickness ta2 of the second conductive member 22 is different from the third conductive member thickness ta3 of the third conductive member 23.

In the sensor 110, the first resistance member 11 may be one of the plurality of resistance elements included in the bridge circuit. The second resistance member 12 may be one of the plurality of resistance elements included in the bridge circuit. The third resistance member 13 may be one of the plurality of resistance elements included in the bridge circuit.

As shown in FIG.3B, the first detection section 11E may include a first conductive layer 15a and a second conductive layer 15b. The first resistance member 11 is provided between the first conductive layer 15a and the second conductive layer 15b. As shown in FIG.4B, the second detection section 12E may include a third conductive layer 15c and a fourth conductive layer 15d. The second resistance member 12 is provided between the third conductive layer 15c and the fourth conductive layer 15d. As shown in FIG.5B, the third detection section 13E may include a fifth conductive layer 15e and a sixth conductive layer 15f. The third resistance member 13 is provided between the fifth conductive layer 15e and the sixth conductive layer 15f. By providing these conductive layers, for example, warpage of the detection section can be suppressed.

The embodiments may include the following Technical proposals:

### (Technical proposal 1)

A sensor, comprising:
an element section; and
a controller,
the element section including:
   a first element including a first detection section,
   a second element including a second detection section, and
   a third element including a third detection section,
the first detection section including a first resistance member and a first conductive member,
the second detection section including a second resistance member and a second conductive member, and
the third detection section including a third resistance member,
the controller being configured to perform a first operation, a second operation, and a third operation,
in the first operation, the controller being configured to apply a first voltage to the first conductive member, not to apply the first voltage to the second conductive member, and to detect a first value corresponding to a first difference between a first electrical resistance of the first resistive member and a second electrical resistance of the second resistive member,
in the second operation, the controller being configured to apply a second voltage to the first conductive member and to detect a second value corresponding to a second difference between the first electrical resistance and a third electrical resistance of the third resistive member, and
in the third operation, the controller being configured to apply a third voltage to the second conductive member and to detect a third value corresponding to a third difference between the second electrical resistance and the third electrical resistance.

### (Technical proposal 2)

The sensor according to Technical proposal 1, wherein
the controller is configured to output a fourth value obtained by calculation using the first value, the second value, and the third value.

### (Technical proposal 3)

The sensor according to Technical proposal 2, wherein
the fourth value includes a value corresponding to a concentration of each of a plurality of types of detection target substances included in a detection target.

### (Technical proposal 4)

The sensor according to Technical proposal 2, wherein
the calculation includes correction of an other influence of a detection target.

### (Technical proposal 5)

The sensor according to any one of Technical proposals 1-4, wherein
the first voltage is different from the second voltage.

### (Technical proposal 6)

The sensor according to any one of Technical proposals 1-5, wherein
a first power consumption is calculated from the first voltage and a first conductive member resistance value of the first conductive member,
a second power consumption is calculated from the second voltage and the first conductive member resistance value,
the first power consumption is smaller than the second power consumption, and
a ratio of a first absolute value of a first power consumption difference between the first power consumption and the second power consumption to the first power consumption is 0.4 or more.

### (Technical proposal 7)

The sensor according to any one of Technical proposals 1-5, wherein
a first power consumption is between a third power consumption and a second power consumption,
the first power consumption is calculated from the first voltage and a first conductive member resistance value of the first conductive member,
the second power consumption is calculated from the second voltage and the first conductive member resistance value, and
the third power consumption is calculated from the third voltage and a second conductive member resistance value of the second conductive member.

### (Technical proposal 8)

The sensor according to any one of Technical proposals 1-7, wherein
a third electric resistance value of the third resistance member at a first temperature is different from a first electric resistance value of the first resistance member at the first temperature, and
the third electrical resistance value is different from a second electrical resistance value of the second resistance member at the first temperature.

### (Technical proposal 9)

The sensor according to Technical proposal 8, wherein
the first temperature is lower than a temperature of the first detection section in the first operation,
the first temperature is lower than a temperature of the first detection section in the second operation, and
the first temperature is lower than a temperature of the second detection section in the third operation.

### (Technical proposal 10)

The sensor according to Technical proposal 8, wherein
an absolute value of a difference between the third electrical resistance value and the first electrical resistance value is larger than an absolute value of a difference between the first electrical resistance value and the second electrical resistance value.

### (Technical proposal 11)

The sensor according to any one of Technical proposals 1-10, wherein
an electrical resistance of the second conductive member is different from an electrical resistance of the first conductive member.

### (Technical proposal 12)

The sensor according to any one of Technical proposals 1-5, wherein
a second temperature of the first resistance member when the first electric power is supplied to the first conductive member is higher than a first temperature,
a second change rate difference is smaller than a first change rate difference,
the first change rate difference is an absolute value of a difference between a first change rate of the first electrical resistance with respect to temperature at the first temperature and a second change rate of the second electrical resistance with respect to temperature at the first temperature, and
the second change rate difference is an absolute value of a difference between a second temperature state change rate of the first electrical resistance with respect to temperature at the second temperature and the second change rate.

### (Technical proposal 13)

The sensor according to Technical proposal 12, wherein
a third temperature of the first resistance member when a second electric power is supplied to the first conductive member is higher than the first temperature,
a fourth change rate difference is smaller than a third change rate difference,
the third change rate difference is an absolute value of a difference between the first change rate and a third change rate of the third electrical resistance with respect to temperature at the first temperature, and
the fourth change rate difference is an absolute value of a difference between a third temperature state change rate of the first electrical resistance with respect to temperature at the third temperature and the third change rate.

### (Technical proposal 14)

The sensor according to Technical proposal 13, wherein
a first electric resistance value of the first resistance member at the first temperature is different from a second electric resistance value of the second resistance member at the first temperature,
the first electric resistance value is different from a third electric resistance value of the third resistance member at the first temperature, and
the second electrical resistance value is different from the third electrical resistance value.

### (Technical proposal 15)

The sensor according to any one of Technical proposals 1-14, wherein
the first resistance member and the second resistance member satisfy at least one of a first condition, a second condition, or a third condition,
in the first condition, a first length of the first resistance member is different from a second length of the second resistance member,
in the second condition, a first width of the first resistance member is different from a second width of the second resistance member,
in the third condition, a first thickness of the first resistance member is different from a second thickness of the second resistance member,
the first resistance member and the third resistance member satisfy at least one of a fourth condition, a fifth condition, or a sixth condition,
in the fourth condition, the first length is different from a third length of the third resistance member,
in the fifth condition, the first width is different from a third width of the third resistance member,
in the sixth condition, the first thickness is different from a third thickness of the third resistance member,
the second resistance member and the third resistance member satisfy at least one of a seventh condition, an eighth condition, or a ninth condition,
in the seventh condition, the second length is different from the third length,
in the eighth condition, the second width is different from the third width, and
in the ninth condition, the second thickness is different from the third thickness.

### (Technical proposal 16)

The sensor according to any one of Technical proposals 1-14, wherein
the first element includes:
   a first base region,
   a first fixed portion fixed to a part of the first base region, and
   a first support portion supported by the first fixed portion, the first support portion supporting the first detection portion,
a first gap is provided between another part of the first base region and the first detection section,
the second element includes:
   a second base region,
   a second fixed portion fixed to a part of the second base region, and
   a second support portion supported by the second fixing portion, the second support portion supporting the second detection portion,
a second gap is provided between another part of the second base region and the second detection section,
the third element includes:
   a third base region,
   a third fixed portion fixed to a part of the third base region, and
   a third support portion supported by the third fixing portion, the third support portion supporting the third detection portion,
   a third gap is provided between another part of the third base region and the third detection section.

### (Technical proposal 17)

The sensor according to Technical proposal 16, wherein
the first base region is a part of a base,
the second base region is another part of the base, and
the third base region is another part of base.

### (Technical proposal 18)

The sensor according to Technical proposal 1 or 2, wherein
in the first operation, the first electric resistance responds to a detection target around the element section, and
a change rate of the first electrical resistance with respect to a detection target in the first operation is higher than a change rate of the second electrical resistance with respect to the detection target in the first operation.

### (Technical proposal 19)

The sensor according to Technical proposal 1 or 2, wherein
in the second operation, the first electric resistance responds to a detection target around the element section, and
a change rate of the first electrical resistance with respect to the detection target in the second operation is higher than a change rate of the third electrical resistance with respect to the detection target in the second operation.

### (Technical proposal 20)

The sensor according to Technical proposal 1 or 2, wherein
in the third operation, the second electric resistance responds to a detection target around the element section, and
a change rate of the second electrical resistance with respect to the detection target in the third operation is higher than a change rate of the third electrical resistance with respect to the detection target in the third operation.

According to the embodiment, a sensor capable of improving characteristics can be provided.

In the specification of the application, "perpendicular" and "parallel" refer to not only strictly perpendicular and strictly parallel but also include, for example, the fluctuation due to manufacturing processes, etc. It is sufficient to be substantially perpendicular and substantially parallel.

Hereinabove, exemplary embodiments of the invention are described with reference to specific examples. However, the embodiments of the invention are not limited to these specific examples. For example, one skilled in the art may similarly practice the invention by appropriately selecting specific configurations of components included in sensors such as, bases, detection sections, controller, etc., from known art. Such practice is included in the scope of the invention to the extent that similar effects thereto are obtained.

Further, any two or more components of the specific examples may be combined within the extent of technical feasibility and are included in the scope of the invention to the extent that the purport of the invention is included.

Moreover, all sensors practicable by an appropriate design modification by one skilled in the art based on the sensors described above as embodiments of the invention also are within the scope of the invention to the extent that the purport of the invention is included.

Various other variations and modifications can be conceived by those skilled in the art within the spirit of the invention, and it is understood that such variations and modifications are also encompassed within the scope of the invention.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the invention as defined by the appended claims.

## Claims

1. A sensor, comprising:
an element section; and
a controller,
the element section including:
a first element including a first detection section,
a second element including a second detection section, and
a third element including a third detection section,
the first detection section including a first resistance member and a first conductive member,
the second detection section including a second resistance member and a second conductive member, and
the third detection section including a third resistance member,
the controller being configured to perform a first operation, a second operation, and a third operation,
in the first operation, the controller being configured to apply a first voltage to the first conductive member, not to apply the first voltage to the second conductive member, and to detect a first value corresponding to a first difference between a first electrical resistance of the first resistive member and a second electrical resistance of the second resistive member,
in the second operation, the controller being configured to apply a second voltage to the first conductive member and to detect a second value corresponding to a second difference between the first electrical resistance and a third electrical resistance of the third resistive member, and
in the third operation, the controller being configured to apply a third voltage to the second conductive member and to detect a third value corresponding to a third difference between the second electrical resistance and the third electrical resistance.

2. The sensor according to claim 1, wherein
the controller is configured to output a fourth value obtained by calculation using the first value, the second value, and the third value.

3. The sensor according to claim 2, wherein
the fourth value includes a value corresponding to a concentration of each of a plurality of types of detection target substances included in a detection target.

4. The sensor according to claim 2, wherein
the calculation includes correction of an other influence of a detection target.

5. The sensor according to any one of claims 1-4, wherein
the first voltage is different from the second voltage.

6. The sensor according to any one of claims 1-5, wherein
a first power consumption is between a third power consumption and a second power consumption,
the first power consumption is calculated from the first voltage and a first conductive member resistance value of the first conductive member,
the second power consumption is calculated from the second voltage and the first conductive member resistance value, and
the third power consumption is calculated from the third voltage and a second conductive member resistance value of the second conductive member.

7. The sensor according to any one of claims 1-6, wherein
a third electric resistance value of the third resistance member at a first temperature is different from a first electric resistance value of the first resistance member at the first temperature, and
the third electrical resistance value is different from a second electrical resistance value of the second resistance member at the first temperature.

8. The sensor according to claim 7, wherein
the first temperature is lower than a temperature of the first detection section in the first operation,
the first temperature is lower than a temperature of the first detection section in the second operation, and
the first temperature is lower than a temperature of the second detection section in the third operation.

9. The sensor according to claim 8, wherein
an absolute value of a difference between the third electrical resistance value and the first electrical resistance value is larger than an absolute value of a difference between the first electrical resistance value and the second electrical resistance value.

10. The sensor according to any one of claims 1-9, wherein
an electrical resistance of the second conductive member is different from an electrical resistance of the first conductive member.

11. The sensor according to any one of claims 1-10, wherein
the first resistance member and the second resistance member satisfy at least one of a first condition, a second condition, or a third condition,
in the first condition, a first length of the first resistance member is different from a second length of the second resistance member,
in the second condition, a first width of the first resistance member is different from a second width of the second resistance member,
in the third condition, a first thickness of the first resistance member is different from a second thickness of the second resistance member,
the first resistance member and the third resistance member satisfy at least one of a fourth condition, a fifth condition, or a sixth condition,
in the fourth condition, the first length is different from a third length of the third resistance member,
in the fifth condition, the first width is different from a third width of the third resistance member,
in the sixth condition, the first thickness is different from a third thickness of the third resistance member,
the second resistance member and the third resistance member satisfy at least one of a seventh condition, an eighth condition, or a ninth condition,
in the seventh condition, the second length is different from the third length,
in the eighth condition, the second width is different from the third width, and
in the ninth condition, the second thickness is different from the third thickness.

12. The sensor according to any one of claims 1-10, wherein
the first element includes:
a first base region,
a first fixed portion fixed to a part of the first base region, and
a first support portion supported by the first fixed portion, the first support portion supporting the first detection portion,
a first gap is provided between another part of the first base region and the first detection section,
the second element includes:
a second base region,
a second fixed portion fixed to a part of the second base region, and
a second support portion supported by the second fixing portion, the second support portion supporting the second detection portion,
a second gap is provided between another part of the second base region and the second detection section,
the third element includes:
a third base region,
a third fixed portion fixed to a part of the third base region, and
a third support portion supported by the third fixing portion, the third support portion supporting the third detection portion,
a third gap is provided between another part of the third base region and the third detection section.

13. The sensor according to claim 12, wherein
the first base region is a part of a base,
the second base region is another part of the base, and
the third base region is another part of base.

14. The sensor according to claim 1 or 2, wherein
in the first operation, the first electric resistance responds to a detection target around the element section, and
a change rate of the first electrical resistance with respect to a detection target in the first operation is higher than a change rate of the second electrical resistance with respect to the detection target in the first operation.

15. The sensor according to claim 1 or 2, wherein
in the second operation, the first electric resistance responds to a detection target around the element section, and
a change rate of the first electrical resistance with respect to the detection target in the second operation is higher than a change rate of the third electrical resistance with respect to the detection target in the second operation.
